# EUROPÄISCHE PATENTANMELDUNG

(11) **EP 3 553 162 A1**
(43) Veröffentlichungstag der Anmeldung: **16.10.2019**
(21) Anmeldenummer: 19168457.0
(22) Anmeldetag: 10.04.2019
(51) Int. Cl.: C12M 1/107, C02F 9/00, C12M 1/00, C12M 1/26

(54) **VERFAHREN, ANLAGE UND SYSTEM ZUR GÄRRESTAUFBEREITUNG**

(30) Priorität: 13.04.2018 DE 102018108822; 14.04.2018 DE 102018003054; 17.08.2018 DE 102018120117
(71) Anmelder: Niersberger Wohn- und Anlagenbau GmbH & Co. KG, 91052 Erlangen (DE)
(72) Erfinder: BÖHLERT, Torsten, 39167 Hohe Borde OT Niederndodeleben (DE)
(74) Vertreter: Sperschneider, Alexandra

(57) **Zusammenfassung**

Es werden ein Verfahren, eine Anlage, ein System und die Verwendung dieser zur Aufbereitung von Gärresten beschrieben, die bei der Vergärung zur Biogaserzeugung anfallen. Die anfallenden Gärreste einer vorgelagerten Gäreinrichtung werden einer weiteren Nachgäreinrichtung zugeführt, wobei ein Nachgären der Gärreste erfolgt. Danach werden diese Gärreste einer Separiereinrichtung zugeführt, in der die Feststoffe von einem Zentrat der Gärreste getrennt werden. Das Zentrat gelangt anschließend in ein erstes Belebungsbecken, in dem das Zentrat durch einen Belebtschlammprozess mittels Bakterien biologisch gereinigt wird. In einer belüfteten Phase der Reinigung wird Sauerstoff und in einer unbelüfteten Phase wird eine externe Kohlenstoffquelle zugeführt. Danach wird der Belebtschlamm einem ersten Nachklärbecken zugeführt. Das Klarfiltrat wird daraus abgeführt und einer Kleinbelebungsanlage mit integriertem zweitem Nachklärbecken zugeführt, wobei durch einen weiteren Belebtschlammprozess mittels Bakterien eine zusätzliche biologische Reinigung und Durchmischung des Klarfiltrats unter Zuführung von Sauerstoff erfolgt. Anschließend wird das geklärte Abwasser einem dritten Nachklärbecken zugeführt, in dem wenigstens ein Fällungsmittel und/oder Reaktionsmittel dem geklärten Abwasser zugeführt werden.

## Beschreibung

Es werden ein Verfahren, eine Anlage und ein System zur Gärrestaufbereitung sowie die Verwendung dieser zur Aufbereitung von Gärresten beschrieben, die bei der Vergärung zur Biogaserzeugung anfallen.

### Hintergrund

Biogas ist ein Gasgemisch, das vor allem aus Methan besteht. Das methanhaltige Gas entsteht, wenn Biomasse unter Sauerstoffabschluss abgebaut wird. Mikroorganismen, sogenannte Archaebakterien, sind dafür zuständig.

Das Biogas kann in Gasbrennern oder Motoren zur Wärme-, Strom- und Kraftbereitstellung eingesetzt werden. Eine weitere Option sind Biomethanaufbereitungsanlagen, welche die Qualität des erzeugten Biogases auf das Niveau von Erdgas anheben und in das Erdgasnetz einspeisen.

Die dafür notwendige Verfahrenstechnik ist weitgehend vorhanden und ist auf zahlreichen Anlagen im Einsatz. Durch die Verabschiedung des Erneuerbare-Energien-Gesetzes (EEG) wurde der Anreiz für den Bau von landwirtschaftlichen Biogasanlagen auf Güllebasis geschaffen. Gleiches gilt für Abfallvergärungsanlagen, welche ebenfalls gezielt über das EEG gefördert werden.

In den Biogas- und Abfallvergärungsanlagen werden unterschiedliche Biomassen zur Vergärung eingesetzt.

In den Biogasanlagen werden vorrangig Energiepflanzen wie Mais, Zuckerrüben, Gras, Getreideganzpflanzensilage (GPS), Getreide usw. vergoren. Zusätzlich sind tierische Exkremente wie Schweine- oder Rindergülle, Dung, Putenmist oder Hühnertrockenkot für die Erzeugung von Biogas erlaubt und werden auch zum Erlangen des sogenannten Güllebonus eingesetzt.

In den Abfallvergärungsanlagen werden Abfälle aus kommunalen Entsorgungsbetrieben, wie Inhalte der grünen oder braunen Tonne, sowie Lebensmittel- und/oder Schlachtabfälle angenommen.

Diese sogenannten Substrate (insbesondere Energiepflanzen und Abfälle) stellen allein aufgrund ihrer unterschiedlichen Zusammensetzung eine große Herausforderung für eine Gärrestaufbereitung dar. Neben der unterschiedlichen Konsistenz der Stoffe liegen auch die relevanten Parameter für die Klassifizierung als Wirtschaftsdünger bzw. als Gärrestprodukt weit auseinander. Besonders sind hier die Elemente Stickstoff, Phosphor sowie viele Schwermetallverbindungen zu nennen.

### Stand der Technik

Derzeit sind zur Aufbereitung der bei der Biogaserzeugung anfallenden Gärreste, insbesondere zur Minimierung bzw. Verarbeitung von Gärresten, folgende Verfahren bekannt:
- Separation - Trennung der festen Phase vom Gärrest und somit Erzielung einer Volumenreduzierung der flüssigen Lagerung
- Bandtrockner - Trocknung der separierten Feststoffe und somit Minimierung der festen Gärreste
- Solares Verfahren - Trocknung der separierten Feststoffe durch Sonnenenergie
- Stripverfahren - Reduzierung der Stickstofffrachten im flüssigen Gärrest
- Vakuumverdampfung - Verdampfung der flüssigen Phase des Gärrestes
- Membranverfahren - Komplettaufbereitung der flüssigen Phase des Gärrestes

Die bekannten Verfahren weisen jedoch verschiedene Nachteile auf. Zum einen ermöglichen die Verfahren nur eine teilweise Aufbereitung, wobei ein nicht aufbereiteter Rest übrig bleibt. Zum anderen sind die Verfahren sehr zeit- und energieintensiv.

Membranverfahren weisen zudem den Nachteil auf, dass die verwendeten Membranen - in Abhängigkeit des Verschmutzungsgrades des zu reinigenden Gärrests - häufig ausgetauscht werden müssen.

Damit das aus der Aufbereitung gewonnene Abwasser eingeleitet werden kann, bspw. in ein Kanalsystem einer Gemeinde, müssen verschiedene Verordnungen und Richtlinien beachtet und umgesetzt werden. Hierzu zählen die Wasserrahmenrichtlinie (WRRL), das Wasserhaushaltsgesetz (WHG), die Grundwasserverordnung (GrwV) und die Einleitwerte der Abwasserverordnung (AbwV).

Initiiert durch die Novelle des EEG 2014, fortgeführt durch das EEG 2017 und unter Berücksichtigung der Düngemittelverordnung 2017 befindet sich dabei die Biogasbranche im Wandel. Für viele Biogas- und Abfallvergärungsanlagen ist ein wirtschaftlicher Betrieb unter diesen Voraussetzungen nur dann möglich, wenn sie eine vollständige Nutzung der ihnen zur Verfügung stehenden Ressourcen erzielen können.

Im Fokus vieler Anlagen wird dabei die Verwertung der bei der Biogaserzeugung anfallenden Gärprodukte stehen. Mit der Düngemittelverordnung 2017 sind folgende Eckpunkte geschaffen worden, welche sich unmittelbar auf den Betrieb einer Biogasanlage auswirken:
- Lagerkapazität von 6 Monaten für flüssige Wirtschaftsdünger und Gärprodukte
- Lagerkapazität von 9 Monaten ab dem Jahr 2020 bei > 3/ha GV oder ohne eigene Aufbringungsflächen
- Verkürzung der Sperrfrist ab Ernte der Hauptkultur (mit Ausnahmeregelungen aber auch mit Begrenzung max. Aufbringmengen von Ammoniumstickstoff bzw. Gesamtstickstoff)
- Sperrfrist für Grünland und mehrjährigem Feldfutterbau vom 01.11. bis zum 31.01.
- Betriebliche Aufbringungsgrenze von 170 kg N/ha

Somit müssen neue und langfristig sichere Wege für die Verwendung der anfallenden Gärreste gefunden werden. Folgende Bedingungen sollen dabei berücksichtigt werden:
- Veredelung der Gärreste zu Gärprodukten (Wertschöpfung durch Vermarktung von Düngemitteln)
- Reduzierung der Gärrestmengen hinsichtlich der bestehenden Flächenknappheit
- Reduzierung der Gärrestmengen hinsichtlich Lagerung, Transport und Ausbringung
- Verringerung der Umweltbelastung (z.B. Nitratgehalt im Grundwasser)

Die Erfüllung der Vorgaben der vorstehend genannten Verordnungen und Richtlinien ist Voraussetzung dafür, dass das Abwasser eingeleitet werden kann. Die bereits angeführten Verfahren erfüllen jedoch nicht oder nur unzureichend die Vorgaben.

### Aufgabe

Bei Anlagen zur Erzeugung von Biogas wäre es daher wünschenswert eine Möglichkeit zu schaffen, die dabei entstehenden Gärreste so aufzuarbeiten, dass neben einer Feststoffphase, die bspw. als Düngemittel verwendet werden kann, einleitfähiges Abwasser bereitgestellt wird. Die bei der Aufbereitung entstehende flüssige Phase als Endprodukt soll daher so weit aufbereitet sein, dass sämtliche Vorgaben hinsichtlich der Schadstoffbelastung eingehalten werden.

Es besteht daher die Aufgabe darin, ein Gärrestaufbereitungskonzept anzugeben, in dem flüssiger Gärrest (Abwasser) vorflutfähig aufbereitet und die entwässerten Rückstände als Gärprodukt abgesetzt werden können.

Ferner ist es Aufgabe, die Gärrestaufbereitung gegenüber bekannten Verfahren zu verbessern und insbesondere die Stickstofffrachten zu reduzieren sowie eine Anlage und ein System anzugeben, welche zur Gärrestaufbereitung dienen.

### Lösung

Die vorstehend genannte Aufgabe wird durch ein Verfahren zur Aufbereitung von Gärresten gelöst, die bei der Vergärung zur Biogaserzeugung anfallen, aufweisend wenigstens folgende Verfahrensschritte:
- Zuführen der anfallenden Gärreste aus einer vorgelagerten Gäreinrichtung zur Biogaserzeugung einer weiteren Nachgäreinrichtung,
- Nachgären der anfallenden Gärreste in der weiteren Nachgäreinrichtung,
- Zuführen der Gärreste aus der weiteren Nachgäreinrichtung einer Separiereinrichtung,
- Trennen der Feststoffe von dem Zentrat der Gärreste in der Separiereinrichtung,
- Zuführen des Zentrats einem Gärrestlager,
- Zuführen des Zentrats einem ersten Belebungsbecken,
- biologische Reinigung des Zentrats durch einen Belebtschlammprozess mittels Bakterien, wobei eine Nitrifikation und Denitrifikation bei einer intermittierenden Belüftung erfolgen, und wobei in der belüftete Phase Sauerstoff und in der unbelüfteten Phase eine externe Kohlenstoffquelle zugeführt werden,
- Zuführen des Belebtschlammes einem ersten Nachklärbecken, in dem eine Trennung des Belebtschlamms durch Separation erfolgt,
- Abführen des Klarfiltrats,
- Zuführen des Klarfiltrats einer Kleinbelebungsanlage mit integriertem zweitem Nachklärbecken, wobei durch einen weiteren Belebtschlammprozess mittels Bakterien eine biologische Reinigung und Durchmischung des Klarfiltrats unter Zuführung von Sauerstoff erfolgt,
- Zuführen des geklärten Abwassers einem dritten Nachklärbecken, in dem wenigstens ein Fällungsmittel und/oder Reaktionsmittel dem geklärten Abwasser zugeführt werden.

Die vorstehenden Verfahrensschritte ermöglichen durch die Abfolge eine Aufbereitung von Gärresten, wobei als eines der Endprodukte einleitfähiges Abwasser bereitgestellt wird.

Die Zugabe der Kohlenstoffquelle und des Fällmittels und/oder des Reaktionsmittels unterstützen die Aufbereitung und sind für die Bereitstellung von einleitfähigem Abwasser wesentlich.

Die einzelnen Verfahrensschritte sind in ähnlicher Form bereits aus dem Stand der Technik bekannt. Die Reihenfolge der einzelnen Reinigungs- und Aufbereitungsschritte ermöglicht jedoch eine nicht vorhersehbare Verbesserung der Qualität des geklärten Abwassers hinsichtlich der Entfernung von Kohlenstoffverbindungen und Nährstoffen, wie Stickstoff und Phosphor.

Insbesondere wird ein Verfahren angegeben, welches eine Aufbereitung von Gärresten ermöglicht und dabei ohne mechanische Filtereinrichtungen und Membrane auskommt. Hierdurch entfallen das häufige Austauschen von Filtern bzw. Membranen und/oder die Reinigung von solchen Einrichtungen.

Die bei dem Verfahren festen, entwässerten Rückstände können als Düngemittel verwendet oder der Landwirtschaft oder Kompostierung zugeführt werden.

In dem Gärrestlager kann der darin aufgenommene Gärrest für einen Zeitraum von mehreren Tagen verweilen. Es muss dabei auf eine ausreichende Durchmischung geachtet werden, damit es zu keinen Feststoffablagerungen und Faulprozessen im Gärrestlager kommt.

Die Separiereinrichtung kann eine Zentrifuge sein, in der durch Rotation die Feststoffe des Gärrests von der flüssigen Phase, dem Zentrat, getrennt werden.

Bei der 2-Phasen-Trennung über die Separiereinrichtung werden die flüssige und die feste Phase voneinander getrennt. Die Feststoffteilchen können sich hierbei aufgrund der höheren Dichte an einer Trommelwand sammeln und werden mit Hilfe einer Förderschnecke zu Austragsöffnungen transportiert. Gleichzeitig kann die geklärte Flüssigkeit an der Schnecke entlang in Flüssigkeitsaustragszone fließen.

Das Belebtschlammverfahren im ersten Belebungsbecken ist ein Verfahren zur biologischen Abwasserreinigung. Dabei wird das Zentrat durch die Stoffwechsel-Aktivität von aeroben chemoorganoheterotrophen Mikroorganismen, dem sogenannten Belebtschlamm, weitestgehend von organischen Verunreinigungen befreit, also gereinigt. Vorteilhaft sind die allgemeine Verwendbarkeit und die gute Reinigungswirkung für das Zentrat zur Verminderung der Gehalte an Schwebstoffen, dem Chemischen Sauerstoffbedarf (CSB), dem Biochemischen Sauerstoffbedarf (BSB5) und den Stickstoffverbindungen (N).

Bei dem Prozess handelt es sich um eine Schwachlastreinigung mit Schwerpunkt der Nitrifikation und Denitrifikation zur weitestgehenden Stickstoffelimination. Diese Art der Reinigung resultiert aus dem Überschuss der Nährstoffe im Vergleich zu verfügbaren Kohlenstoffverbindungen.

Während der biologischen Reinigung im ersten Belebungsbecken kann gegen den Abfall des pH-Wertes des zu reinigenden Zentrats und zur Erhöhung des Puffervermögens ein alkalischer Hilfsstoff zugeführt werden. Als alkalischer Hilfsstoff kann bspw. Natronlauge zugeführt werden.

Zusätzlich kann während der biologischen Reinigung im ersten Belebungsbecken dem Zentrat bspw. Phosphorsäure zugeführt werden, um ein bestimmtes Verhältnis von BSB zu Phosphor - je nach Beschaffenheit des Gärrests - zu erreichen und aufrechtzuerhalten. Bspw. kann das Verhältnis des biochemischen Sauerstoffbedarfs (BSB) zu Phosphor 100:1 betragen. Ist das Zentrat arm an Phosphor, so muss dann Phosphor, bspw. in Form von Phosphorsäure, zugeführt werden.

In den Nachklärbecken erfolgt eine Trennung der festen Stoffe durch Sedimentation. Die von der flüssigen Phase gelösten Stoffe setzen sich dabei am Boden der Nachklärbecken ab.

Das erste Nachklärbecken ist Bestandteil des Belebtschlammverfahrens und erfüllt folgende Aufgaben:
- Optimale Abtrennung der Feststoffe (Schlamm) vom gereinigten Wasser, geringe Konzentrationen im Ablauf (Feststoffe),
- Trennung des Belebtschlamm-Wasser-Gemisches,
- Eindickung des Schlammes, um einen hochkonzentrierten Rücklaufschlamm und Überschussschlamm bereitzustellen, und
- Speicherungsfunktion zur Vermeidung des Ausschwemmens der Biomasse aus dem System, insbesondere bei erhöhter hydraulischer Beschickung.

Das Nachklärbecken basiert auf dem Prinzip der Separation. Das Abwasser-Belebtschlamm-Gemisch gelangt aus dem Belebungsbecken in das Nachklärbecken. Der im Wasser enthaltene Schlamm setzt sich ab (Sedimentation) und wird im unteren Bereich gesammelt und eingedickt.

Dem Klarfiltrat kann vor und/oder während des Einleitens in die Kleinbelebungsanlage zusätzlich ein Fällungsmittel zugeführt werden, welches das Absetzen der Stoffe beschleunigt.

Der bei der biologischen Reinigung im ersten Belebungsbecken, im ersten Nachklärbecken, im zweiten Nachklärbecken und im dritten Nachklärbecken anfallende Überschussschlamm kann der vorgelagerten Gäreinrichtung zugeführt werden. Anschließend wird dieser Überschussschlamm wieder in den Prozess eingebracht, wobei eine Trennung der Feststoffe von dem Zentrat in der Separiereinrichtung erfolgt und anschließend die weiteren Verfahrensschritte durchlaufen werden. Es bleibt somit kein Rest zurück, der nicht weiter verarbeitet werden kann.

Den Gärresten kann vor der Trennung der Feststoffphase von dem Zentrat ein Flockungsmittel zugeführt werden, welches die Trennung unterstützt.

Zusätzlich kann bei der Trennung der Feststoffphase von dem Zentrat Verdünnungswasser zugeführt werden. Das Verdünnungswasser kann aus vorgelagerten Prozessen der Biogaserzeugung stammen und wird vorteilhaft weiter verwendet. Ein vorgelagerter Prozess kann bspw. das Waschen von Ausgangsstoffen für die Biogaserzeugung umfassen. Ferner kann das Verdünnungswasser von einem Regenrückhaltebecken stammen, welches den Niederschlag einer Biogasanlage und/oder Gärresteaufbereitungsanlage sammelt.

Bei der intermittierenden Belüftung können die belüftete Phase und die unbelüftete Phase jeweils 1 bis 2 Stunden, vorzugsweise 1,5 Stunden, aufrechterhalten werden.

Als Fällungsmittel können Aluminiumchlorid (AlCl₃) oder Polyaluminiumhydroxidchlorid (PAC) zugeführt werden. Zusätzlich oder alternativ können als Flockungsmittel Polymere zugeführt werden. Polyaluminiumhydroxidchlorid (PAC) eignet sich insbesondere als kombiniertes Flockungs- und Fällungsmittel.

Als Kohlenstoffquelle kann insbesondere Glycerin zugeführt werden.

Die vorstehend genannte Aufgabe wird auch durch eine Anlage zur Aufbereitung von Gärresten gelöst, wobei die Anlage dazu ausgelegt und ausgebildet ist, ein Verfahren zur Gärrestaufbereitung gemäß einer der vorstehenden Verfahren durchzuführen, mindestens aufweisend
- eine weitere Nachgäreinrichtung,
- eine Separiereinrichtung zur Trennung der festen Phase vom Zentrat des Gärrests,
- ein Gärrestlager,
- ein erstes Belebungsbecken zur biologischen Reinigung des Zentrats,
- ein erstes Nachklärbecken zur Trennung des Belebtschlamms,
- eine Kleinbelebungsanlage mit integriertem zweiten Nachklärbecken zur weiteren biologischen Reinigung, und
- ein drittes Nachklärbecken zur Absetzung des Belebtschlamms.

Die Anlage kann neben den wesentlichen Komponenten zur Gärrestaufbereitung auch Bestandteile umfassen, die zur Biogaserzeugung dienen.

Die vorstehend genannte Aufgabe wird auch durch ein System zur Aufbereitung von Gärresten gelöst, welches Komponenten zur Durchführung der Verfahrensschritte der vorstehenden Verfahren aufweist.

Schlüsselkomponenten der Gärrestaufbereitung bilden wenigstens die Separiereinrichtung, die bspw. mindestens eine Dekanterzentrifuge umfasst, zur gemeinsamen Entwässerung der Gärreste und/oder Überschussschlämme, und das Belebtschlammverfahren in der intermittierenden Fahrweise zur Entfernung hauptsächlich der verbliebenen gelösten Kohlenstoffverbindungen und Nährstoffe (wenigstens N und/oder P).

Die festen entwässerten Rückstände können bspw. in einem Container gesammelt und der Landwirtschaft oder Kompostierung zugeführt werden. Der geklärte flüssige Gärrest (Abwasser) wird in die Vorflut abgeleitet.

Die Separiereinrichtung stellt in diesem Konzept eine Schlüsseltechnologie dar. Durch die Separiereinrichtung wird der Gärrest in Feststoff und Flüssigkeit getrennt. Der Feststoff kann in erdiger Form mit einem Trockensubstanzgehalt von bspw. 25 - 35 Gewichts-% anfallen. Dieser feste Gärrest kann direkt als Wirtschaftsdünger verwendet oder durch Kompostierung, Trocknung und Pelletierung veredelt werden.

Die flüssige Phase, das sogenannte Zentrat, wird dann weitergeleitet zur biologischen Abwasserreinigung. Die "Reinheit" des Zentrates, ausgedrückt durch den Abscheidegrad, ist entscheidend für den Erfolg der Abwasserreinigung. Bei dem Trennungsprozess werden eine Vielzahl von Hauptnährstoffgehalten wie Gesamtstickstoff, Ammoniumstickstoff, Phosphor usw. durch den Austrag mittels Feststoff bis zu 25 Gewichts-% reduziert. Die Nährstoffe müssen dann nicht mehr weiter behandelt werden, sondern verbleiben im festen Gärrest als Düngemittelbestandteil.

Das saubere Zentrat wird im Gärrestlager gepuffert und der anschließenden Abwasserreinigung im Belebungsbecken zugeführt. Das Zentrat aus dem Gärrestlager kann mit einer kontinuierlichen Menge in das Belebungsbecken gepumpt werden.

Der Belebtschlammprozess mit den beiden Phasen Nitrifikation und Denitrifikation findet in einem Behälter statt, in dem das sogenannte intermittierende Belüftungsverfahren erfolgt.

In der belüfteten Phase wird der Ammonium-Stickstoff entsprechend der Reaktion zum Nitrit und weiter zum Nitratstickstoff umgewandelt:

NH₄⁺ + O₂ + 2HCO₃⁻ -> NO₃⁻ + 2CO₂ + H₂O

In der unbelüfteten Phase erfolgen die Zugabe einer externen C-Quelle (bspw. Glyzerin) und eine intensive Durchmischung des Abwasser(Zentrat)-Belebtschlamm-Gemisches, damit eine Denitrifikation entsprechend der folgenden Gleichung stattfinden kann:

2NO₃⁻ + 2H⁺ + 2,5C organisch -> N₂ + H₂O + 2,5CO₂

Die Zugabe des Glyzerins gleicht das zur Denitrifikation benötigte Kohlenstoffdefizit im Verhältnis BSB5/NH4-N = 4 aus.

Das Nachklärbecken lässt sich in 4 Zonen einteilen:
- die Klarwasserzone
- die Trennzone
- die Speicherzone
- die Eindick- bzw. Räumzone.

Der Schlamm aus der Eindickzone wird abgezogen und gelangt als Rücklaufschlamm zurück in das erste Belebungsbecken. Das geklärte Wasser wird aus der Klarwasserzone mit verschiedenen Techniken abgezogen und zur nächsten Reinigungsstufe (Kleinbelebungsanlage) weitergeleitet.

Als letzte Reinigungsstufe ist das dritte Nachklärbecken (Fällungsbecken) vorgesehen, wobei zur weiteren Reduzierung des CSB-Wertes und ggf. des Phosphorgehaltes dem gereinigten Abwasser ein Fällungsmittel hinzugegeben wird. Der dabei entstehende Fällschlamm setzt sich im Becken ab und wird mit Hilfe von Pumpen aus dem Becken abgezogen.

Im Weiteren ist es möglich, zusätzliche Verfahrensschritte einzufügen oder der Gärrest- bzw. Abwasseraufbereitung nachzuschalten. Bspw. können zusätzliche Maßnahmen ergriffen werden, wenn die ermittelten Werte nach der Feststofftrennung (bspw. mittels eines Dekanters) nicht erreicht werden. In diesem Fall kann eine separate Verfahrensstufe für die Entfernung von Phosphor zum Einsatz kommen, da die Fällung des Phosphors rein über Fällungsmittel nicht mehr praktikabel sein kann.

Um den Phosphor in der flüssigen Phase für die Landwirtschaft verfügbar zu machen, kann bspw. das sogenannte AirPrex®-Verfahren angewendet werden.

Mit dem AirPrex®-Verfahren lässt sich die biologische Phosphat-Elimination verbessern. Der Gärrest wird nach einem Fermenter in ein AirPrex®-Reaktorsystem geführt und dort einer Luftstrippung unterzogen. Durch das Ausgasen von CO₂ steigt der pH-Wert des Schlammes deutlich an. Gleichzeitig führt die Zugabe von Magnesium-Fällsalzen zur Bildung und Ausfällung von Magnesium-Ammonium-Phosphat (MAP). Die Kristalle integrieren sich homogen in die Schlammmatrix. Das gewonnene MAP, das auch als Struvit bezeichnet wird, kann anschließend als Dünger verwendet werden.

Phosphorhaltige Dünger können somit einfach erhalten werden, wobei einerseits der Phosphor aus dem Gärrest verarbeitet und andererseits den immer geringer werdenden, verfügbaren Phosphormengen Rechnung getragen werden.

Das AirPrex®-Verfahren kann die Rentabilität einer Anlage zusätzlich signifikant erhöhen. Die Komplettlösung für die Verarbeitung von Phosphor (Struvit-Problem) sorgt für reibungslose Abläufe und gute Resultate in der Phosphat-Elimination. Folgende Verbesserungen können mit diesem Verfahren erzielt werden:

### - Weniger Phosphat-Rückbelastung

Die Reduktion von Ortho-Phosphaten im Schlamm verringert das Potenzial für weitere Kristallisationen und mindert die Rückbelastung einer Anlage.

### - Weniger Polymerverbrauch

Durch die Reduktion der Ortho-Phosphate kann sich der Verbrauch an zusätzlichen Flockungshilfsmitteln um bis zu 30 Prozent reduzieren.

### - Geringere Entsorgungskosten

Die geringere Wasserbindung steigert den Trockensubstanzgehalt und verringert so das Entsorgungsvolumen und damit die Entsorgungskosten von Gärresten.

### - Bessere Ablaufqualität

Das in einem Fermenter rückgelöste Ortho-Phosphat kann zu mehr als 90 Prozent im MAP gebunden werden. So lassen sich Schlammentwässerung und Ablaufqualität verbessern.

### - Geringere Betriebskosten

Die MAP-Kristallisation kann in einem dafür konzipierten Reaktor durchgeführt und damit vorgezogen werden. So können Betriebsstörungen in den anschließenden Anlagen ausgeschlossen werden, wodurch sich die Betriebskosten reduzieren.

Die hierin beschriebenen Verfahren, Anlagen und Systeme weisen eine Vielzahl an Vorteilen auf. So sind die allgemeine Verwendbarkeit und die gute Reinigungswirkung für Abwässer zur Verminderung der Gehalte an Schwebstoffen, dem Chemischen Sauerstoffbedarf (CSB), dem Biochemischen Sauerstoffbedarf (BSB5) und den Stickstoffverbindungen (N) als vorteilhaft zu betrachten. Zudem liegen eine einfache Bedienung und ein hoher Automatisierungsgrad vor. Die Nährstoffe sind im festen Gärrest konzentriert, wodurch die Transport- und Ausbringkosten reduziert sind. Feste Gärreste können zudem je nach Bedarf weiter veredelt werden, z.B. als Agrardünger, Einstreu, Kompost usw. Es können die restriktiven Vorschriften der Düngeverordnung 2017 eingehalten werden und es ergibt sich eine Verringerung der Grund- und Oberflächengewässerbelastung durch Stickstoff, Nitrat und Phosphor. Die Lagerhaltung von Gärrest wird zusätzlich reduziert, wobei bis zu 80% des Gärrestes zu sauberem Wasser werden und somit vorflutfähig sind. Das hierin beschriebene Konzept eignet sich im Weiteren für die Voll- oder Teilreinigung von Güllen aus der Tierhaltung, von Gärresten sowie von Industrieabwasser, wobei niedrige spezifische Reinigungskosten bei großen Mengen Gärrest anfallen.

Weitere Vorteile, Merkmale und Ausgestaltungsmöglichkeiten ergeben sich aus der nachfolgenden Figurenbeschreibung von nicht einschränkend zu verstehenden Ausführungsbeispielen.

### Kurzbeschreibung der Zeichnungen

In den Zeichnungen zeigt:
- Fig. 1: eine schematische Darstellung der Schritte im Vergährungsprozess;
- Fig. 2: ein schematisches Ablaufdiagramm der Reinigungs- und Aufbereitungsschritte;
- Fig. 3: eine schematische Darstellung von Komponenten einer Anlage zur Gärrestaufbereitung;
- Fig. 4: eine schematische Darstellung eines Reaktorsystems zur Durchführung eines Airplex®-Verfahrens;
- Fig. 5: eine schematische Darstellung der Umsätze bei der Gärrestaufbereitung;
- Fig. 6: eine schematische Schnittansicht einer Zentrifuge;
- Fig. 7: eine beispielhafte Darstellung einer Kleinbelebungsanlage (KBA);
- Fig. 8: ein Blockschaltbild eines Nachgärers 3;
- Fig. 9 - 28:: Tabellen 1 - 20.

In den Figuren mit gleichen Bezugszeichen versehene Teile entsprechen im Wesentlichen einander, sofern nichts anderes angegeben ist. Darüber hinaus wird darauf verzichtet Bestandteile zu zeigen und zu beschreiben, die nicht wesentlich zum Verständnis der hierin offenbarten technischen Lehre sind. Im Weiteren werden nicht für alle bereits eingeführten und dargestellten Elemente die Bezugszeichen wiederholt, sofern die Bestandteile selbst und deren Funktion bereits beschrieben wurden oder für einen Fachmann bekannt sind.

### Ausführliche Beschreibung eines Ausführungsbeispiels

Im Folgenden werden das Konzept einer Anlage zur Gärrestaufbereitung sowie die bei der Gärrestaufbereitung durchgeführten Verfahrensschritte anhand von Beispielen beschrieben.

Es werden hierzu drei bestehende Anlage zur Biogaserzeugung herangezogen, um die Ausgangslage von Gärresten hinsichtlich deren Bestandteile und Konzentration aufzuzeigen. Im Weiteren wird anhand eines Beispiels aufgezeigt, wie eine Anlage aufgebaut werden muss, um die erforderliche Reinheit von einleitfähigem Wasser zu erreichen.

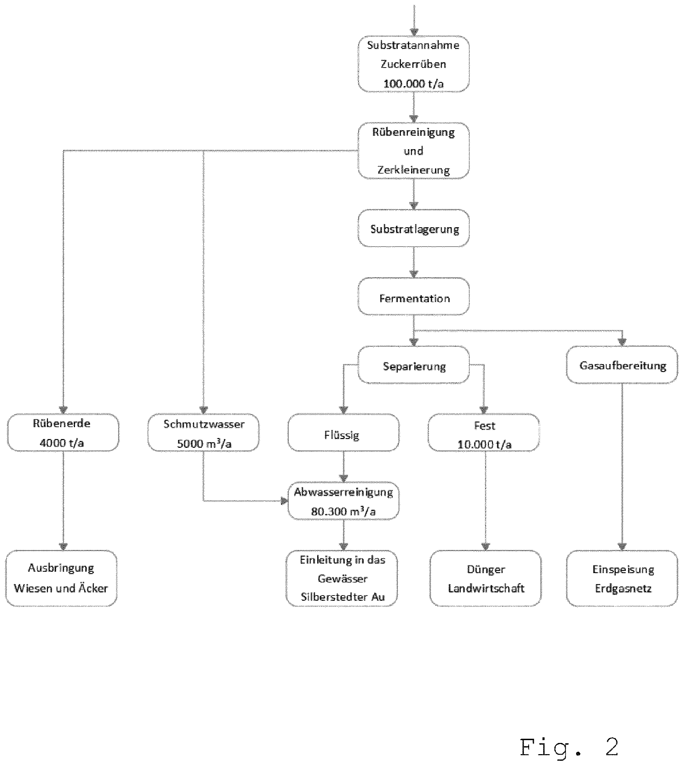

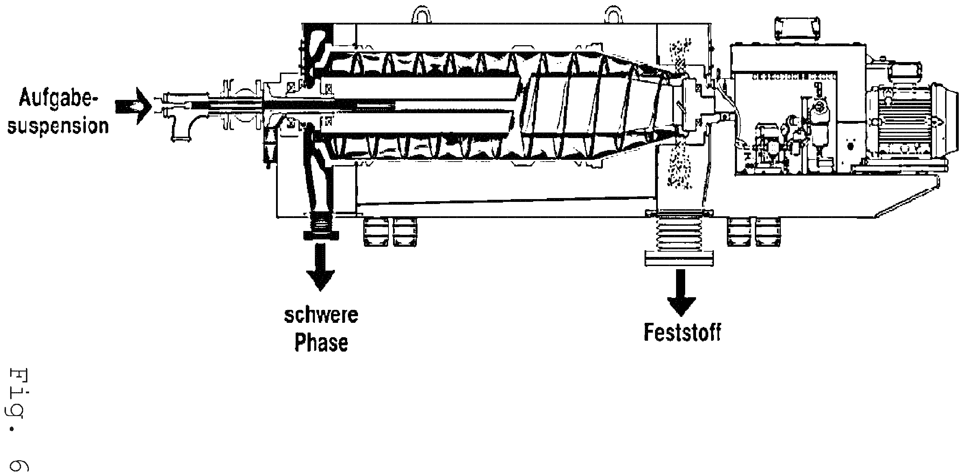

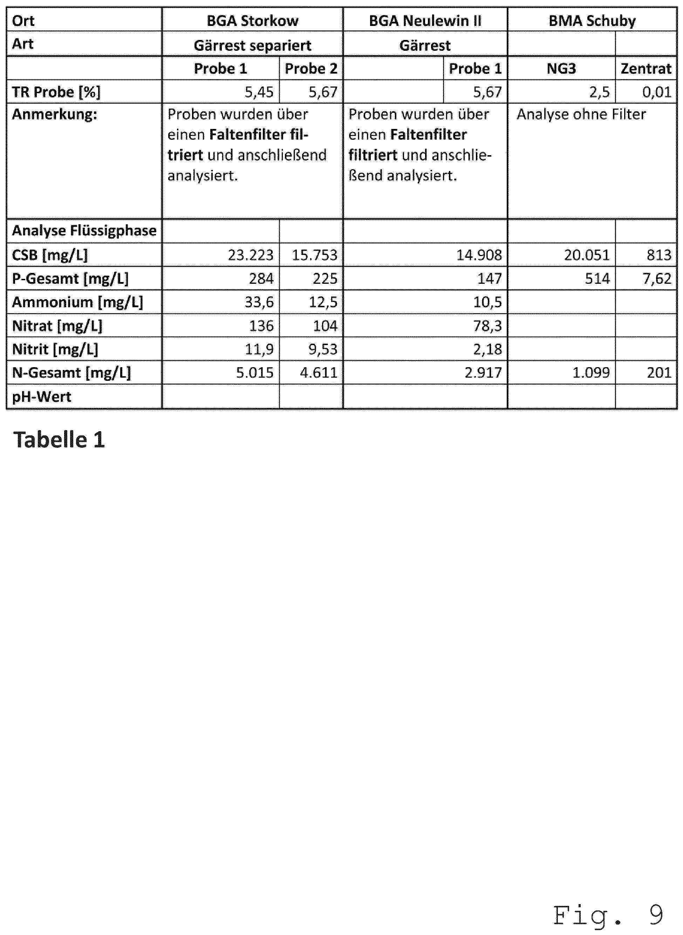

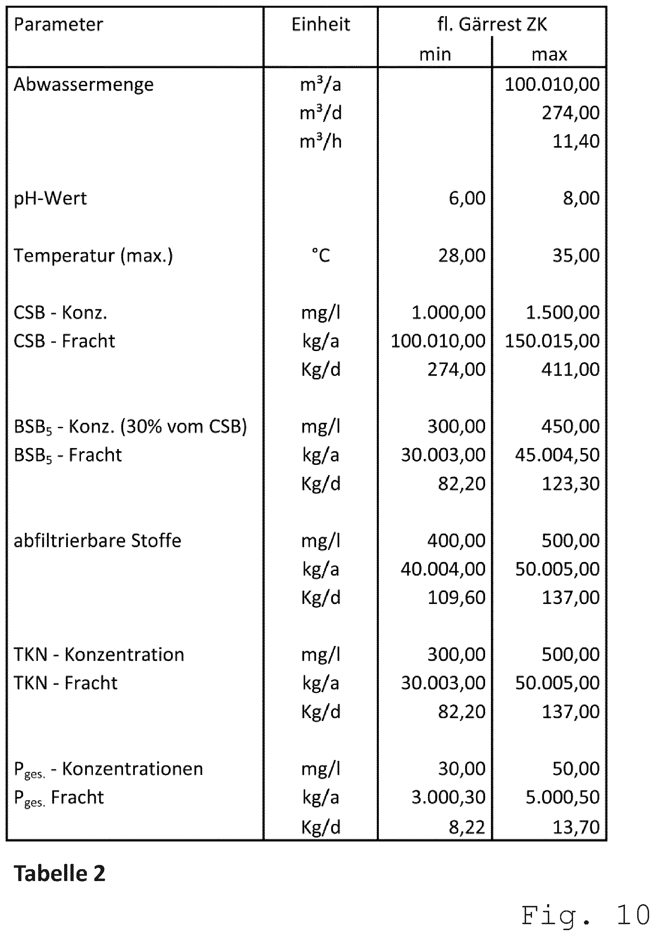

In den Fig. 2 bis 8 ist eine Anlage zur Erzeugung von Biogas und zur Aufbereitung der dabei entstehenden Gärreste mit wesentlichen Bestandteilen gezeigt. Die Fig. 9 bis 28 zeigen Tabellen, auf die im Folgenden zur Erläuterung des Prinzips der Gärrestaufbereitung anhand von Beispielen hingewiesen wird.

In Fig. 9 sind in der dargestellten Tabelle beispielhaft die Ergebnisse von Gärrestanalysen aus drei typischen Biogasanlagen (BGA Storkow, BGA, Neulewin II, BMA Schuby) dargestellt.

Die BGA Storkow wird vorrangig mit Maissilage und einem sehr hohen Anteil an Hühnertrockenkot (HTK) und Entengülle beschickt. Sie stellt den klassischen Typ einer NawaRo-Anlage (Nachwachsende Rohstoffe) dar, welche einen "Güllebonus" gemäß EEG über den Einsatz von HTK sichert.

Die BGA Neulewin wird mit einem sehr vielfältigen Mix aus nachwachssenden Rohstoffen und Rindergülle sowie -mist beschickt. Diese Anlage gehört zu der Gruppe von Biogasanlagen, die in Deutschland weit verbreitet sind.

Die BMA Schuby wird ausschließlich mit Zuckerrüben gefüttert, eine sogenannte Monovergärung, und repräsentiert einen weiteren Bestandteil der Biogasanlagen in Deutschland.

Die BMA Schuby dient als Basis für die Darstellung des hierin beschriebenen Konzepts der Gärrestaufbereitung, wobei nachfolgend aufgezeigt wird, wie die Anlage aufzubauen ist, um einleitfähiges Abwasser bereitzustellen.

Die analysierten Flüssigphasen der drei Biogasanlagen zeigen die großen Parameterunterschiede in der Nährstoffzusammensetzung insbesondere hinsichtlich Phosphor, Stickstoff und CSB (chemischer Sauerstoffbedarf) auf, welche üblicherweise bei Biogasanlagen vorliegen. Bei Abfallvergärungsanlagen kann von gleichen Verhältnissen ausgegangen werden.

Die bei den drei Biogasanlagen als Gärrest anfallenden Flüssigphasen können in der vorliegenden Zusammensetzung nicht ohne weiteres in die Umwelt eingeleitet werden, so dass eine Aufbereitung der Flüssigphasen erfolgen muss. Hierzu gibt es Voraussetzungen für die Einleitung in öffentliche Gewässer.

Es muss daher geprüft werden, unter welchen Bedingungen das Einleiten von aufbereitetem Abwasser aus der flüssigen Phase des Gärrestes aus der Biogaserzeugung möglich ist. Hierzu müssen vor Allem die WRRL der Europäischen Union, sowie deren Umsetzung in nationales und Landesrecht im Wasserhaushaltsgesetz und gegebenenfalls bereits bestehende Ausnahmetatbestände der Abwasserverordnung beachtet werden.

Das hier beschriebene Gärrestaufbereitungskonzept wird im Wesentlichen durch zwei Punkte beeinflusst, nämlich durch den zum Einsatz kommenden Substratmix für die Biogaserzeugung, welcher die Zusammensetzung des Gärrestes stark beeinflusst und entscheidend für das Endergebnis der Gärrestaufbereitung ist, und durch die Grenzwerte, die für das bei der Gärrestaufbereitung anfallende Abwasser zum Einleiten eingehalten werden müssen.

Das hierin beschriebene Konzept enthält daher unterschiedliche Bausteine, welche ermöglichen, sich den herrschenden Rahmenbedingungen flexibel anzupassen und somit den wirtschaftlichen Erfolg von Biogasanlagen mit Gärrestaufbereitung sowie Gärrestaufbereitungsanlagen im jeweiligen Umfeld zu sichern. Hierfür muss auch eine Anpassung an die Rahmenbedingungen von Biogasanlagen und Gärrestaufbereitungsanlagen erfolgen.

Bei der Auslegung und Gestaltung der einzelnen Komponenten und Verfahrensschritte werden Analyseergebnisse des jeweils vorliegenden Gärrests herangezogen und es werden die Randbedingungen für die Biogasanlage, sowie die eventuellen Einleitwerte für den Vorfluter ermittelt.

### Darstellung einer beispielhaften Anlage

Die BMA Schuby wird als Beispiel zur Darstellung des hierin offenbarten Konzepts zur Gärrestaufbereitung herangezogen. Das Konzept kann auch auf andere Biogasanlagen, wie bspw. die Biogasanlagen Storkow und Neulewin, sowie andere Gärrestaufbereitungsanlagen übertragen werden, wobei entsprechende Modifikationen an den Komponenten und den Verfahrensschritten erforderlich sind. Beispielsweise muss eine Anpassung der zugeführten Stoffe an den jeweiligen Nährstoffgehalt (bspw. Stickstoff, Phosphor) und der Kohlenstoffverbindungen des Gärrests erfolgen. Zudem sind die Mengen an Gärrest wesentlich für die Ausgestaltung einer Gärrestaufbereitungsanlage.

### Biogaserzeugung

Der BMA Schuby werden Zuckerrüben als Ausgangssubstrat für die Biogaserzeugung zugeführt. Anstelle von Zuckerrüben können anderen Anlagen, welche das hierin beschriebene Konzept umsetzen oder aufweisen, andere Substrate für die Biogaserzeugung zugeführt werden.

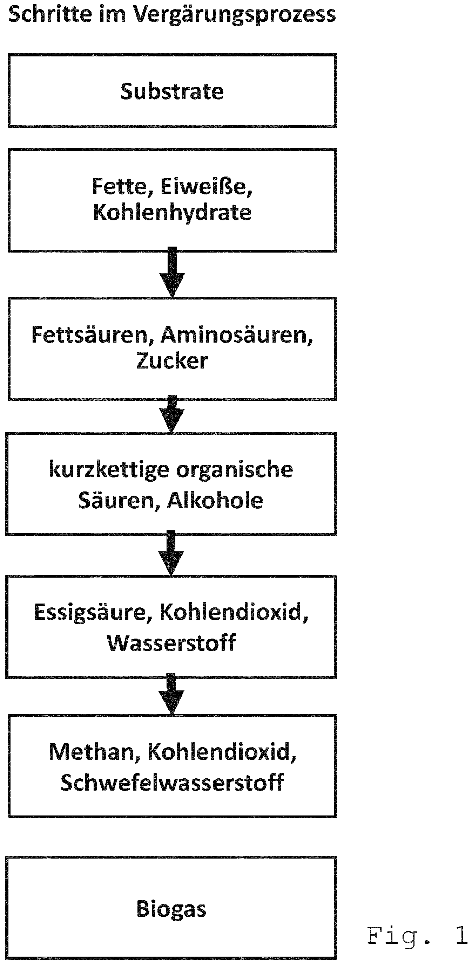

Fig. 1 zeigt schematisch die einzelnen Schritte im Vergärungsprozess.

Für die Vergärung werden als Substrat Zuckerrüben verwendet, wobei eine klassische Monovergärung stattfindet. Die Biogasanlage Schuby kann bspw. für jährlich 100.000 bzw. täglich 274 Tonnen an Substrat ausgelegt sein.

### Rübenannahme - Waschwasserhandhabung

Obwohl der Verschmutzungsgrad von Zuckerrüben, die bereits am Feldrand vorgereinigt werden, im Hinblick auf eventuell sandige und schwachlehmige Erzeugerflächen bereits stark reduziert ist, werden diese dennoch bei Rübenannahme aufbereitet.

In der Rübenannahme werden die Rüben von trockener Erde und Fremdkörpern befreit, in mehreren Reinigungsschritten aufbereitet und letzten Endes zerkleinert bzw. gemust.

Der Schmutzgehalt von angelieferten Rüben kann bspw. zwischen 3% und 8%, bspw. etwa 5 %, der angelieferten Masse annehmen. Im Zuge eines täglichen mehrstündigen Betriebes der Rübenannahme können die angelieferten Zuckerrüben von anhaftender Erde, Schlamm, Steinen und sonstigen Fremdstoffen befreit werden. Die abgeschiedenen Stoffe können gesammelt werden.

In einem Wäscher kann der eigentliche Waschprozess aus dem Waschwasserbehälter vollzogen und trockene anhaftende Erde abgewaschen werden. Die gewaschenen Rüben können über einen Stabförderer, in dem ein Teil des anhaftenden Wassers und Schlamms abtropft, mit einer anhaftenden Restfeuchte von ca. 1% der Gesamtmasse und mit ca. 1 % anhaftender Restverschmutzung dem Zerkleinerer bzw. Muser zugeführt werden.

Das Waschwasser, das abtropfende Wasser und Schlamm können in einen zweigeteilten Waschwasserbehälter fließen. Im ersten Teil können zunächst aufschwimmende Feststoffe, wie Rübenbruch, der im Zuge der mechanischen Reinigung anfällt, und sonstige Feststoffe, wie Rübenkraut, mittels eines Feststoffabscheiders abgezogen werden. Mittels eines freien Überlaufs kann das Waschwasser in den zweiten Teil des Waschwasserbehälters fließen. Hier setzen sich feine Partikel in Form von Schlamm ab und können in regelmäßigen Abständen mit einem externen Gerät entnommen und entsorgt werden.

Das am Waschwasserbehälter abgezogene Rübenkraut und Rübenbruch kann über ein, zwei oder mehrere Förderaggregate ebenfalls dem vorhandenen Rübenzerkleinerer zugeführt werden.

Das Waschwasser aus dem Waschwasserbehälter kann soweit möglich zum Waschen im Kreislauf geführt werden. Bei Bedarf kann Waschwasser aus einem Absetzcontainer oder, falls nur eine unzureichende bevorratete Waschwassermenge vorhanden ist, über einen Brauchwasserbrunnen zugeführt werden. Eventuell überschüssiges Waschwasser kann über einen Schacht einem Pumpwerk während der Rübenannahmekampagne in die Absetzcontainer oder im Bedarfsfall in den Nachgärer 3 abgesenkt werden.

In den Absetzcontainer kann Waschwasser als Vorlagereserve für die Rübenwäsche vorgehalten und im Bedarfsfall der Rübenwäsche zugeführt werden. Hierzu kann zum einem überschüssiges Waschwasser aus dem Waschwasserbehälter und während der Rübenannahmekampagne Niederschlagswasser von der Rübenannahmefläche über ein Pumpwerk zwischengespeichert werden. Bei Überfüllung kann die automatische Absenkung des Waschwassers in den Nachgärer 3 erfolgen.

Außerhalb der Rübenkampagne kann die Rübenannahmefläche gereinigt und das unverschmutzte Niederschlagswasser über das Pumpwerk in ein Regenrückhaltebecken geleitet werden.

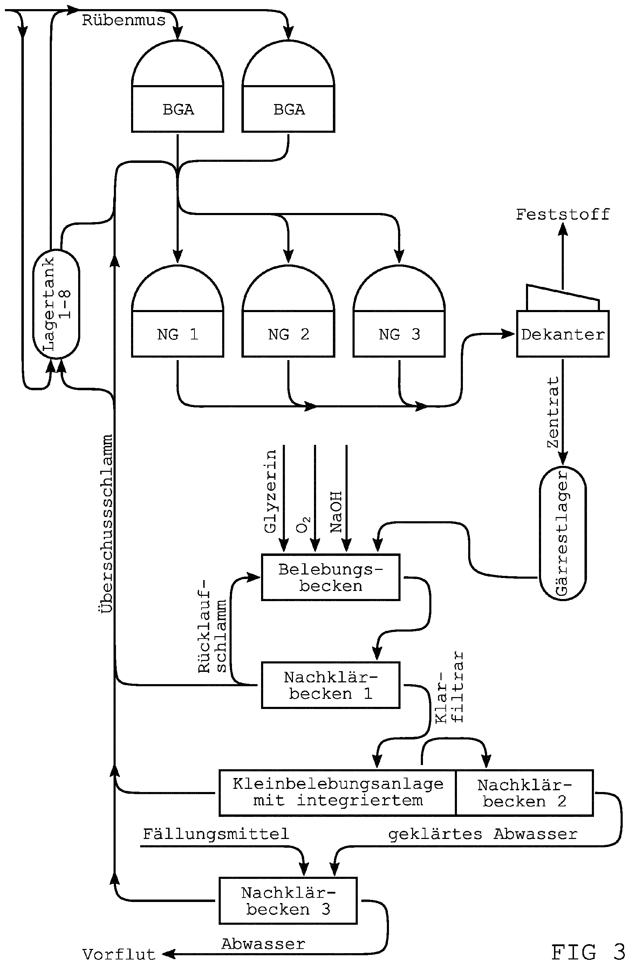

Die Zuckerrüben werden in der Kampagne von der Feldrandmiete zum Biogasanlagengelände transportiert und über die vorhandene Waage erfasst, abgekippt und in die festinstallierte Rübenaufbereitung gefördert. Durch die Rübenaufbereitung werden die auf dem Feld vorgereinigten Rüben noch einmal entsteint, dann gewaschen und gemust. Die in der Rübenannahme anfallenden Rübenspitzen und Rübenkleinteile, können über Fördersysteme der Rübenaufbereitung wieder zugeführt werden. Diese Rückführung verkleinert erheblich die zu entsorgenden Rückstände aus der Rübenreinigung. Über wenigstens eine Pumpe, vorteilhaft wenigstens eine Rachentrichterpumpe, wird das Rübenmus in Lagertanks 1-8 (Fig. 3) gepumpt. Die Durchsatzleistung der Rübenaufbereitung kann 60 Tonnen in der Stunde betragen, so dass am Tag 900 Tonnen Zuckerrüben verarbeitet werden können.

Die Rübenmussilage kann anschließend Fermentern über redundant ausgeführte Pumpwerke zugeführt werden. Die Fermentation weist vorteilhaft zwei Anlagenlinien auf, die jeweils aus einem Hochfermenter und einem zugehörigen Nachgärer aufgebaut sind. Über wenigstens einen Desintegration-Verfahrensschritt, einer Behandlung im Hochspannungsfeld, wird vorhandenes Restgaspotential weiter ausgeschöpft.

Das erzeugte Biogas wird durch wenigstens eine Verdichterstation abgezogen, über wenigstens eine Biogaskühlung zur Abscheidung von Kondensat geleitet und zur Biogasaufbereitungsanlage gefördert, beispielsweise über eine oder mehrere Pumpvorrichtungen und/oder Saugvorrichtungen. Die Biogasaufbereitung bereitet das Biogas über die Abscheidung des verbliebenen Wasserdampfs und der Schlechtgaskomponenten (CO₂, H₂, H₂S) entsprechend auf und übergibt das verbliebene Biomethan mit einem Volumenstrom von 1070 Nm³/h mit einem Methangehalt von mindestens 97% CH₄ an die Biogasmethaneinspeisestation.

### Gärrestaufbereitung

Die im Vergärungsprozess anfallenden Gärreste werden anschließend durch die Gärrestaufbereitungsanlage so aufbereitet, dass das dabei anfallende Abwasser in einen Vorfluter eingeleitet werden kann. Die Gärrestaufbereitungsanlage ist dabei Bestandteil des Konzepts der modifizierten Biogasanlage, wobei einzelne Bestandteile der Gesamtanlage sowohl für die Biogaserzeugung als auch für die Gärrestaufbereitung verwendet werden.

Die eingeleitete Menge an Abwasser wird bspw. auf 80.300 m³/a bilanziert. Auf Grund betriebsbedingter Schwankungen (Schwankung Stoffeigenschaften Zuckerrüben, Diskontinuität Niederschlag zur Waschwasservorhaltung) können sich die jährlich eingeleiteten Abwassermengen leicht verändern.

Die Gärrestentsorgung muss aus Gründen der Betriebssicherheit der industriellen Produktion zu jeder Zeit gewährleistet sein und stellt einen wesentlichen ökologischen und wirtschaftlichen Aspekt beim Betrieb der Biogasanlage dar.

### Ausführungsbeispiel Biogasanlage "Schuby"

Die Biogasanlage Schuby kann täglich bspw. 220 m³ Gärreste mit einem Trockensubstanzgehalt zwischen 2 bis 3% produzieren. Um den Gärrest aufzuarbeiten wird die Anlage modifiziert, wobei Fig. 3 schematisch das Konzept einer Gärrestaufbereitungsanlage mit den wesentlichen Komponenten der Gesamtanlage zeigt. Manche Bestandteile der Gesamtanlage dienen sowohl der Biogaserzeugung als auch der Gärresteaufbereitung.

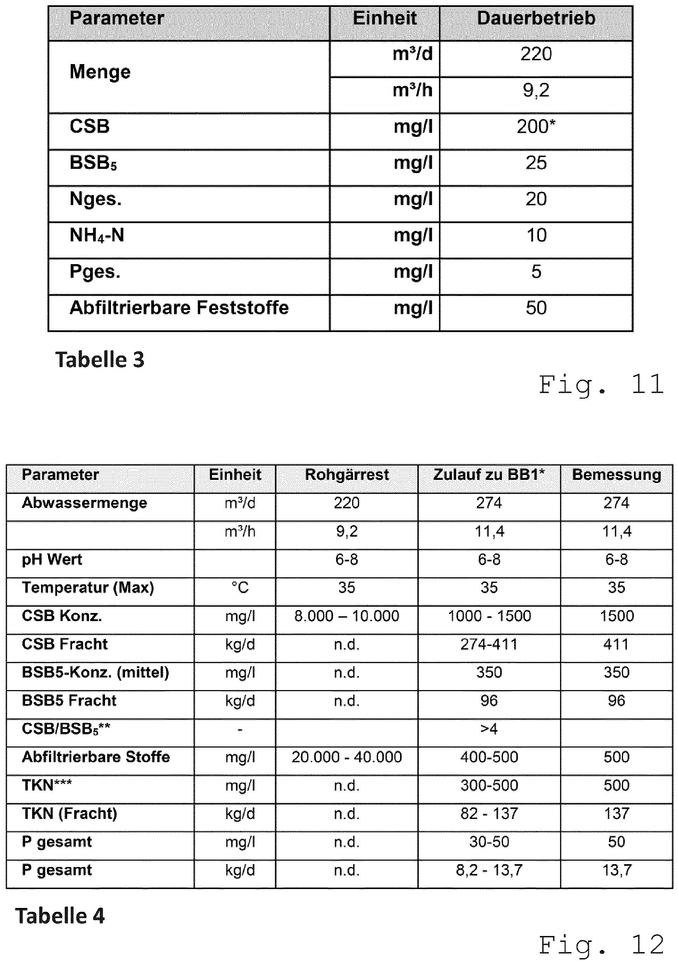

Fig. 11 zeigt in Tabelle 1 die Einleitwerte, die für das Abwasser durch die Gärrestaufbereitung erreicht werden sollen.

Die gezeigten Werte stellen Grenzwerte dar, die nicht überschritten werden dürfen, damit das Abwasser in öffentliche Gewässer eingeleitet werden darf.

Der Ursprung von nichtabbaubaren Substanzen ist bspw. in der Trockensubstanz der Zuckerrübe zu finden. Darunter verbergen sich sog. Lignocellulosen und Hemicellulosen. Beide sind Bestandteile und Strukturelemente pflanzlicher Zellwände, deren Matrix aus fibrillärer, teilweise amorpher Cellulose besteht. Deren biologische Transformationsprodukte in der Abwassertechnik sind Huminsäuren, welche eine Abwasserfärbung von leicht gelb bis dunkelbraun verursachen. Diese Beschaffenheit führt dazu, dass nach der biologischen Reinigung ein persistenter, nicht abbaubarer Anteil als CSB (chemischer Sauerstoffbedarf) im geklärten Abwasser verbleibt. Das Abwasser weist nach einer Aufbereitung stets einen gewissen chemischen Sauerstoffbedarf auf. Als Grenzwert für die Einleitung des Abwassers ist ein CSB-Einleitwert von 200 mg/l angegeben.

Die in der Tabelle von Fig. 12 dargestellten Werte stellen die Bemessungsgrundlage für das weitere nachfolgend beschriebene Verfahren dar. In einem regulären Betrieb der Biogasanlage verbleibt ein Gärrest mit den angegebenen Werten. Der Gärrest muss so behandelt werden, dass die in Tabelle 1 aufgeführten Grenzwerte erreicht werden.

### Gärrestaufbereitung: Abtrennung von überschüssigem Schmutzwasser aus dem Gärrest

In der Fermentation im Rahmen der Rübenmonovergärung entsteht ein nährstoffarmer Gärrest mit einem geringen TS(Trockensubstanz)-Gehalt. Trotz des hohen Abbaugrades der Vergärung verbleibt ein geringer Anteil an biologisch abbaubarem Kohlenstoff im Substrat. Ein weiterer Aufschluss erfolgt durch die Desintegration und nochmaligem biologischen Abbau im Nachgärer 3. Dies führt zu einer weiteren Reduzierung der organischen Belastung und die Nährstoffbelastung nimmt weiter ab und eine mechanische Entwässerung des Gärrestes wird möglich.

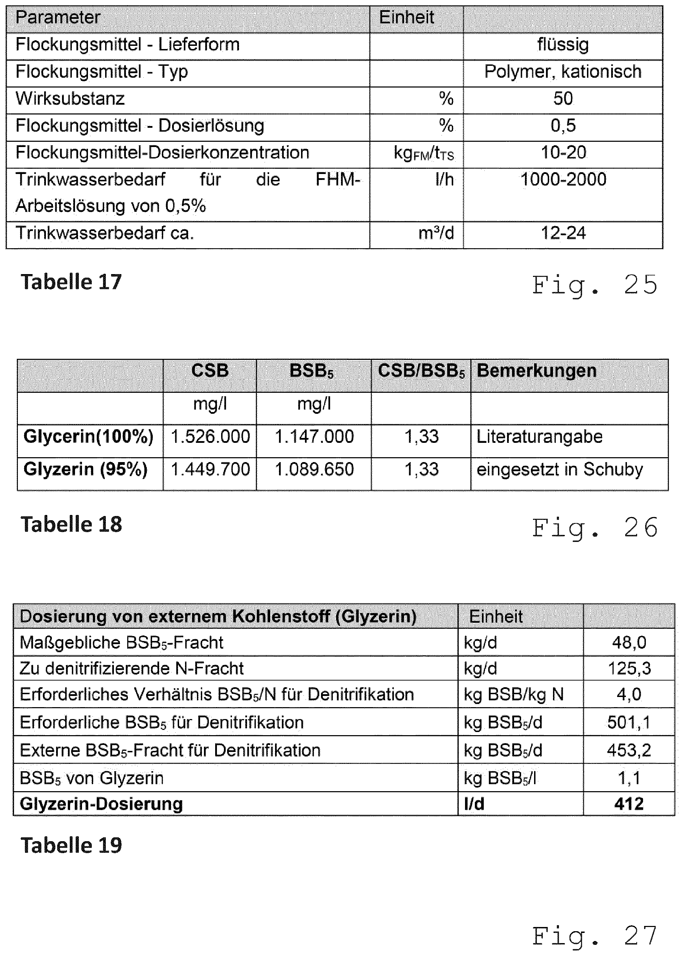

Der Gärrest aus dem Nachgärer 3, bestehend aus Gärrest, Niederschlagswasser, Waschwasser und Überschussschlamm aus der Gärrestaufbereitung, mit einer Gesamtmenge von bspw. 104.230 m³ mit einem maximalen TS-Gehalt von 4% wird über eine Zuführpumpe der Dekanteranlage zur Trennung des Aufgabegutes in Flüssigphase, das sogenannte Zentrat, und die Feststoffphase zugeführt. Um eine bestmögliche Abscheidung der feinen Feststoffpartikel zu erreichen, werden Flockungshilfen (Polymere) zugeführt. Zur Fest-Flüssig-Trennung des Gärrestes vom Schmutzwasser kann die in der Tabelle von Fig. 25 angegebene Menge an Flockungsmittel eingesetzt werden.

Das Gärsubstrat gelangt mit der vorteilhaft angemischten Flockungsmittelsuspension über ein Einlaufrohr in die ebenfalls rotierende Aufgabekammer. Dort wird die Suspension in Drehrichtung beschleunigt und durch Öffnungen in die ebenfalls rotierende Trommel geleitet. Im ersten Abschnitt der Trommel, dem zylindrischen Teil, bewegt sich der Feststoff unter dem Einfluss der Fliehkraft zur Trommelwand. Der herauszentrifugierte Feststoff wird von der Schnecke über den Konus zu den Austragsöffnungen der Trommel transportiert und wird an ein Förderschneckensystem übergeben, das den Feststoff im Wechsel auf zwei Reststoffcontainer verteilt. Bei einem Abscheidegrad von 98% können in dem gezeigten Ausführungsbeispiel bspw. insgesamt ca. 10.030 t fester Gärrest anfallen, der nachfolgend als Dünger auf die Herkunftsflächen ausgebracht werden kann.

Dieser Prozess dient der Entfernung der suspendierten und faserigen Feststoffe (TS = 1,5 bis 2,5%; max. 4%) und wird mittels einer Dekanterzentrifuge mit unterstützender Hilfe der Flockungshilfsmittel durchgeführt.

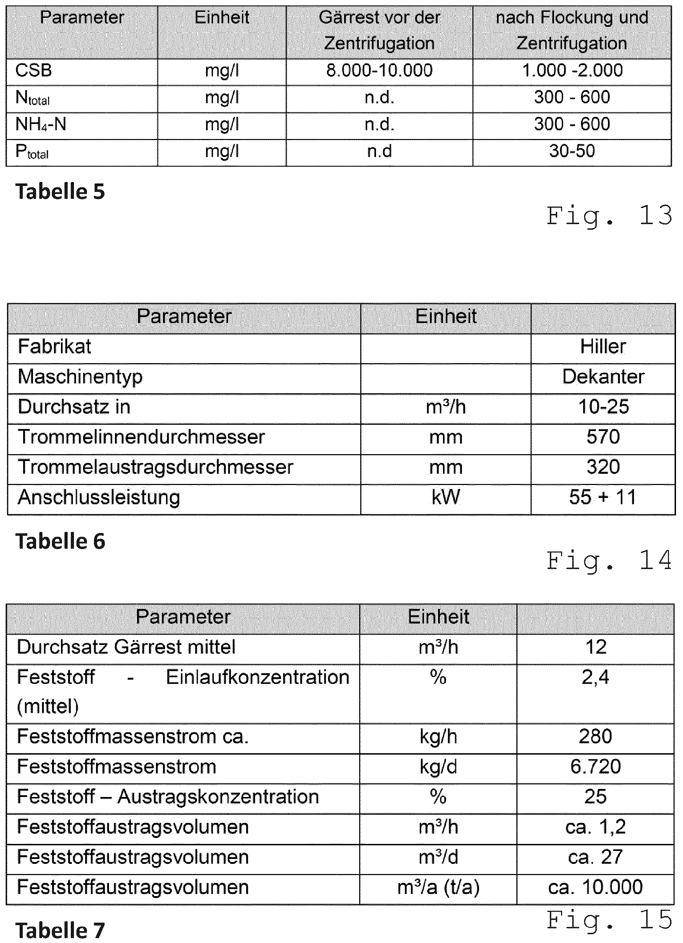

Die Tabelle in Fig. 13 zeigt die Beschaffenheit des Gärrestes, die sich nach der Flockung und Zentrifugation in einem ersten Gärrestaufbereitungsschritt einstellen wird. Zur Bestimmung wurden Versuche durchgeführt.

Für die Feststofftrennung kann eine Zentrifuge zum Einsatz kommen. In der Tabelle von Fig. 14 sind Angaben zur Ausgestaltung der Zentrifuge wiedergegeben. Die Tabelle von Fig. 15 zeigt die Stoffbilanz der Zentrifuge.

Das in der Dekanteranlage anfallende Zentrat läuft in der Trommel über eine Überlaufkante ab und fließt in einen nachfolgenden Gärresteschacht mit Pumpwerk. Das Pumpwerk fördert dieses in den Gärrestbehälter (Gärrestlager - siehe Fig. 3), der als Vorlagetank für das nachfolgende Belebungsbecken dient.

Während des Startens und Stoppens der Dekanteranlage wird das Aggregat mit Frischwasser gespült. Das anfallende Frischwasser wird über den Auslauf am Schneckenförderer in einen eigenen Schmutzwasserschacht geleitet. Dieser nimmt ergänzend das anfallende verschmutzte Niederschlagswasser von der Feststoff-Verladefläche mit auf. Das verschmutzte Wasser wird nachfolgend über eine Tauchmotorpumpe dem Nachgärer 3 zugeführt.

Das Zentrat aus der Gärrest-Feststofftrennung wird dem Gärrestlager zugeführt. Das Volumen des Gärrestlagers beträgt bspw. V = 1.800 m³. Durch das große Volumen wird der Inhalt bei etwaigen Konzentrationsschwankungen homogenisiert. Die rechnerische Verweilzeit des Schmutzwassers beträgt t = 6 d. Es ist dabei auf ausreichende Durchmischung zu achten, um Feststoffablagerungen und Faulprozesse zu vermeiden.

### Gärrestaufbereitung: Behandlung des flüssigen Gärrestes in der Gärrestaufbereitung

Vom Gärrestlagerwird der flüssige Gärrest in das Belebungsbecken gefördert (siehe Fig. 3). In dem Belebungsbecken wird mit Hilfe von spezialisierter Bakterien-Biozönose (Belebtschlammverfahren) das Schmutzwasser biologisch weitgehend gereinigt. Hier findet die intermittierende Nitrifikation und Denitrifikation statt. In der Nitrifikations-Phase wird Sauerstoff über ein Gebläse zugegeben und der biologische Umwandlungsprozess setzt ein. In der Denitritifikations-Phase findet unter Sauerstoffmangel bei Zugabe einer Kohlenstoffquelle der Nitratabbau statt. Für das intermittierende Verfahren wird bspw. ein Wechselintervall von 1,5 Stunden belüftet zu 1,5 Stunde unbelüftet herangezogen. Das geforderte Verhältnis C/N für eine weitgehende Denitrifikation beträgt bspw. 3,5. Da der Bedarf an leicht verfügbarem Kohlenstoff im Vergleich zur bereitgestellten Fracht im Gärrest für eine ausreichende Versorgung der Bakterien nicht ausreicht, ist die Zuführung einer Kohlenstoffquelle erforderlich, die in Form von Glycerin zugeführt wird (siehe Fig. 26). Bei anderen Biogasanlagen oder Gärrestaufbereitungsanlagen können auch andere Kohlenstoffquellen zugeführt werden.

Bei dem Prozess handelt es sich um eine Schwachlastreinigung mit Schwerpunkt der Nitrifikation und Denitrifikation zur weitgehenden Stickstoffelimination. Diese Art der Reinigung resultiert aus dem Überschuss der Nährstoffe (N) im Vergleich zu verfügbaren Kohlenstoffverbindungen.

Der Belebtschlammprozess mit N/DN findet in einem Behälter statt und erfordert eine präzise Steuerung der Belüftungs- und Durchmischungszeiten, d.h. intermittierende Belüftung. In der belüfteten Phase wird der Ammonium-Stickstoff entsprechend der Reaktion zum Nitrit und weiter zum Nitratstickstoff umgewandelt:

NH₄⁺ + O₂ + 2 HC0₃⁻ → NO₃⁻ + 2CO₂ + H₂O

In der ungelüfteten Phase erfolgen die Zugabe der externen C-Quelle (Glyzerin) und eine intensive Durchmischung, damit eine Denitrifikation entsprechend der folgenden Gleichung stattfinden kann:

2NO₃⁻ + 2H⁺ + 2,5C organisch → N₂ + H₂O + 2,5CO₂

Die Zugabe des Glyzerins gleicht den zur Denitrifikation benötigten Kohlenstoffdefizit im Verhältnis BSB4/NH4-N = 4 aus. Die dosierte Menge an Glyzerin sowie seine Spezifikation ist in der Tabelle von Fig. 27 dargestellt.

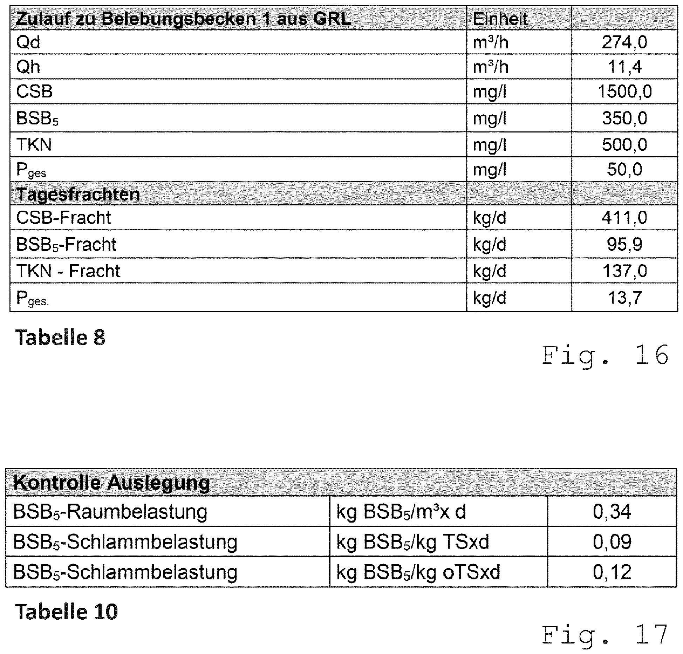

Die in den Fig. 16 und 17 dargestellten Tabellen geben beispielhafte Werte der biologischen Reinigung sowie Mengenangeben wieder.

Da der Gärrest nicht über eine ausreichend hohe Säurekapazität verfügt, kann der pH-Wert infolge der Nitrifikation und Denitrifikation unter 7,0 fallen. In diesem pH-Bereich sind jedoch sowohl die Nitrifikations- und die Sauerstoffausnutzungsrate stark beeinträchtigt.

Voraussetzung für hohe Umsatzgeschwindigkeiten der Nitrifikation ist, dass die gebildeten Säure-Ionen vom Hydrogencarbonatpuffer des Abwassers schnell abgefangen werden. Pro mg abgebautem NH4-N werden bspw. 0,14 mmol (1kg NH4-N - 140 mol) Säurekapazität verbraucht. Bei der Denitrifikation wird das durch die Nitrifikanten gebildete Nitrat zu elementarem Stickstoff abgebaut. Im Gegensatz zur Nitrifikation werden bei der Denitrifikation Säure-Ionen (H+) verbraucht. Theoretisch liefert die Denitrifikation 50 % der zuvor in der Nitrifikation verbrauchten Säurekapazität zurück. Pro mg abgebautem NO3-N werden 0,07 mmol/l Säurekapazität gewonnen. Die tatsächliche Säurekapazität hängt von dem Puffervermögen des jeweiligen Abwassers ab.

In den Fermentern können bspw. TAC-Werte (Carbonat-Pufferkapazität) um 2000 mg/l vorliegen, was einer Säurekapazität von ca. 20 mmol/l entspricht. Der rechnerische Bedarf an Säurekapazität auf Grundlage solcher Werte und einer beispielsweise angenommenen Ablaufkapazität von 1,5 mmol/l aus der 1. Belebungsstufe beträgt 37,5 mmol/l.

Da das Puffervermögen des Gärrestes unzureichend sein kann, müssen Maßnahmen zur Puffererhöhung und gegen den Abfall des pH-Wertes getroffen werden. Dies erfolgt über die Zudosierung von Natronlauge als alkalischen Hilfsstoff.

Da der Gärrest je nach Beschaffenheit des Einsatzstoffes auch arm an Phosphor sein kann und das erforderliche Verhältnis von BSB zu Phosphor 100:1 beträgt, muss diesem ergänzend zur Prozessstabilität Phosphorsäure zugeführt werden.

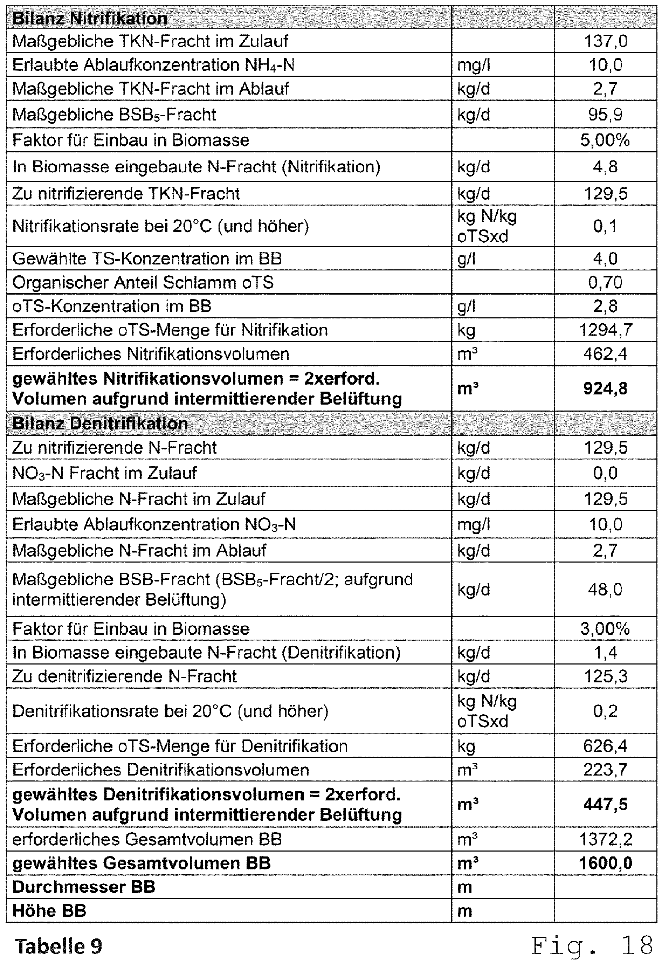

Die Tabelle von Fig. 18 gibt die Ergebnisse einer Kontrolle der biologischen Reinigung wieder.

Vom ersten Belebungsbecken wird der Gärrest zu einem ersten Nachklärbecken in Betonbauweise gefördert. Das erste Nachklärbecken dient der Trennung des Belebtschlammes und des geklärten Schmutzwassers nach dem Belebtschlammprozess.

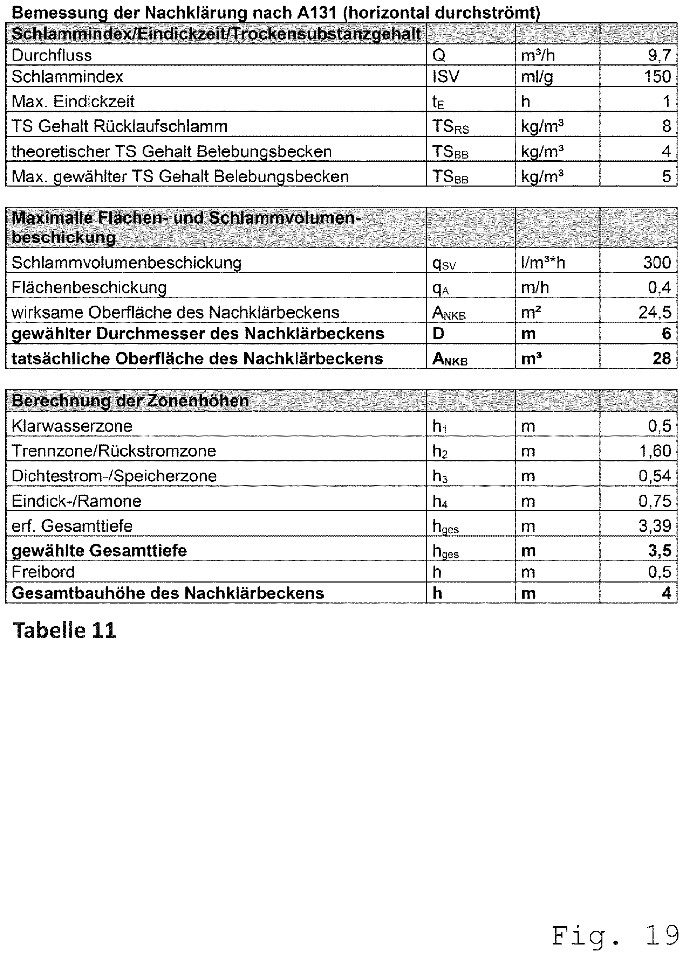

Für die eigentliche Feststofftrennung ist in der der Regel die Fläche maßgeblich, jedoch spielt für eine effiziente Trennung auch die Höhe des Beckens eine Rolle. In der vertikalen Richtung bilden sich im Klärprozess dabei diverse Zonen aus: Klarwasserzone mit mind. h = 0,5 m, Trennzone und Speicher- und Eindickzone, deren Ausdehnung u.a. von den Schlammeigenschaften (SVI) abhängt. In den Tabellen von Fig. 19 sind beispielhafte Angaben einer Dimensionierung des ersten Nachklärbeckens für die Gärrestaufbereitung gezeigt.

Das Becken mit einem Durchmesser von bspw. 6,0 m und einer Höhe von 4,0 m hat bei einer Füllhöhe von 3,5 m ein Füllvolumen von ca. 100 m³. Der Gärrest wird von oben mittig zugeführt. Über Schwerkraft setzen sich Schlammpartikel am Behälterboden ab und werden über einen langsam rotierenden Rundsaugräumer mit Zentralantrieb abgesaugt. Der abgesaugte Schlamm wird zum einem wieder dem Belebungsbecken zur Erhöhung der aktiven Bakterienmasse für Nitrifikation und Denitrifikation zurückgeführt (ca. 1,8-2,0 m³/h) und zum anderen wird der überschüssige Schlamm in den Nachgärer 1 zum biologischen Abbau im Rahmen der Fermentation gepumpt. Im Becken aufschwimmender Schlamm wird abgezogen und ebenfalls dem Nachgärer 1 zugeführt. Der so gereinigte flüssige Gärrest (ca. 220 m³/d), das sogenannte Klarfiltrat, läuft in die Klarwasserrinne über und wird zur weiteren Aufreinigung zu einer Kleinbelebungsanlage mit integrierter Nachklärung (KBA mit Nachklärbecken NKB 2) weitergepumpt.

### Abwasser-/Gärrestaufbereitung: Behandlung des restlichen Abwassers aus der Gärrestaufbereitung

Die Kleinbelebungsanlage kann als Kompaktkleinanlage in Containerbauweise mit integrierter Nachklärung (KBA mit integriertem NKB 2) ausgebildet sein. Diese Belebungsstufe kombiniert das Belebtschlamm- und Tauchkörperverfahren. Neben dem Belebtschlamm entwickelt sich auf rotierenden Tauchkörpern eine sessile Biofilm-Biozönose. Ein Vorteil dieser Kombination liegt in dem hohen Spezialisierungsgrad des Biofilms zum Abbau schwer abbaubarer Substrate.

In der Anlage werden alle vorgenannten Reinigungsschritte und Verfahren bis auf die Denitrifkation abermals durchlaufen. Um eventuell überschüssigen Phosphor, der je nach Beschaffenheit des Einsatzstoffes auftreten kann, fällen und abscheiden zu können, wird dem Klarfiltrat im Zulauf zur KBA ein Fällmittel, wie z.B. Aluminium-Chlorid zugesetzt. Der anfallende Schlamm wird abgezogen und dem Nachgärer 1 zugeführt. Das verbleibende Abwasser wird in den Klarzonen der Nachklärung NKB 2 abgezogen und dem Nachklärbecken NKB 3 zugeführt.

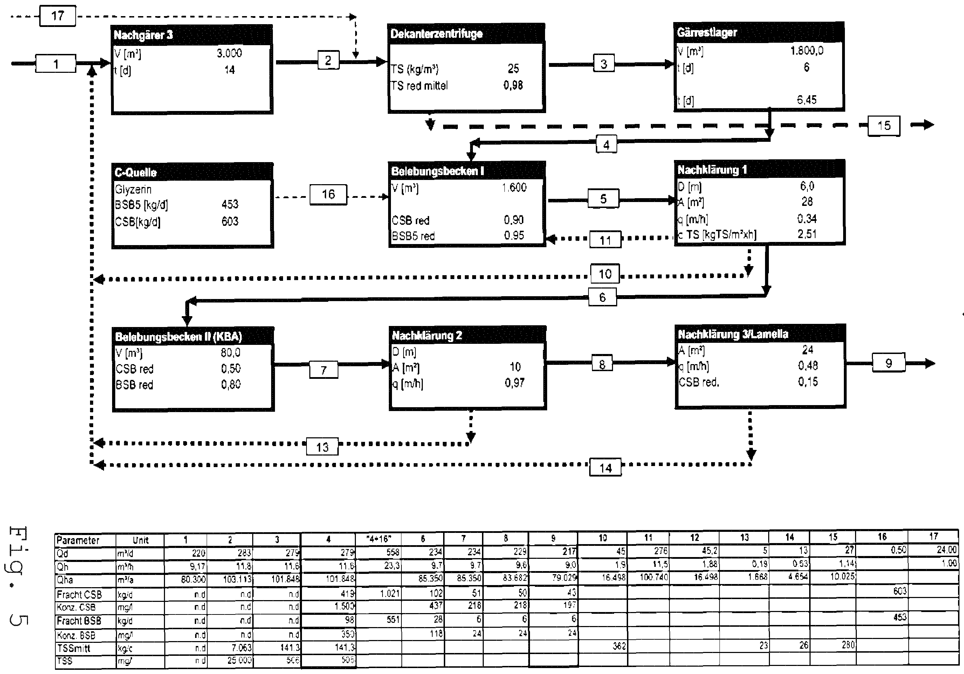

Aufgrund der erwarteten Reinigungsleistung der vorangegangenen Stufen dient diese weitere Reinigungsstufe zur weiteren Reduzierung des "schweren" CSBs hauptsächlich durch spezialisierte Biofilm-Bakterien. Da der Kleinbelebungsanlage verhältnismäßig wenige Substrate zufließen (siehe Fig. 5), ist die interne Rezirkulation des Belebtschlamms nicht notwendig. Der produzierte Überschussschlamm wird am Tiefpunkt der Nachklärsektionen der KBA abgezogen und dem Nachgärer 1 oder Nachgärer 2 zur Nachgärung zugeführt.

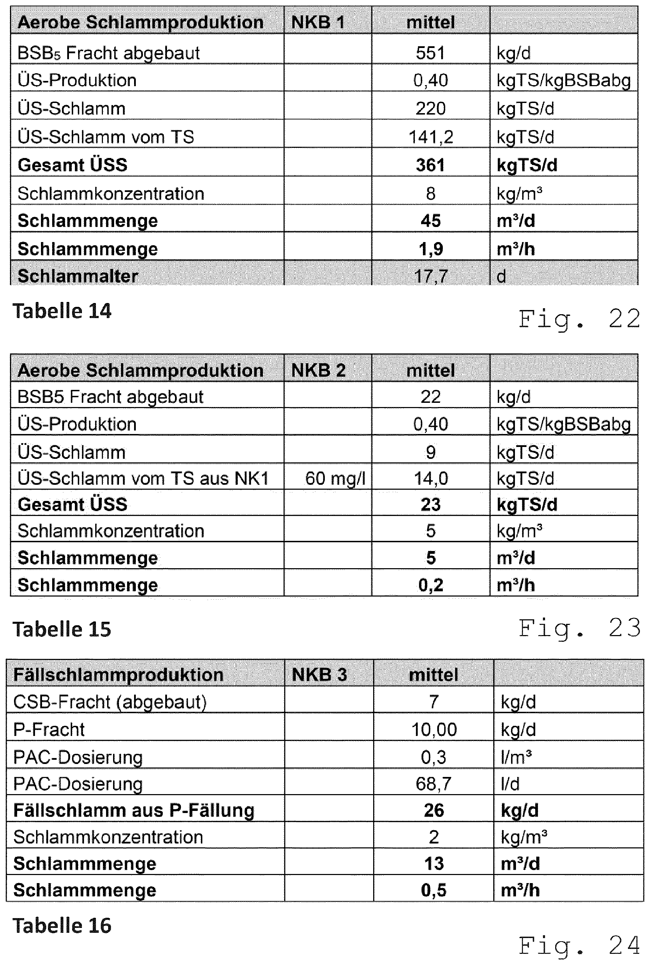

Sämtliche während des biologischen Reinigungsprozesses des Schmutzwassers nach der Abtrennung von Gärrest an den entsprechenden Stufen anfallenden Überschussschlämme können in den Nachgärer 1 oder in den Nachgärer 2 und dann weiter in den Nachgärer 3 geleitet werden. Die rechnerische Menge der in den Nachklärbecken anfallenden Schlämme ist in den Tabellen der Fig. 22, 23 und 24 dargestellt.

Die Überschussschlämme werden in den Nachgärern teilweise anaerob abgebaut und die verbliebenen Reste zusammen mit dem Feststoffen aus dem Vergärungsprozess aus dem Nachgärer 3 der Zentrifuge zur Entwässerung zugeführt. Für die Menge der verbliebenen Feststoffe nach der Entwässerung, die extern entsorgt werden, gilt die Stoffbilanz der Zentrifuge, wie in der Tabelle von Fig. 15 angegeben.

### Abwasseraufbereitung: Behandlung des restlichen Abwassers aus der Gärrestaufbereitung (weitergehende Abwasserbehandlung)

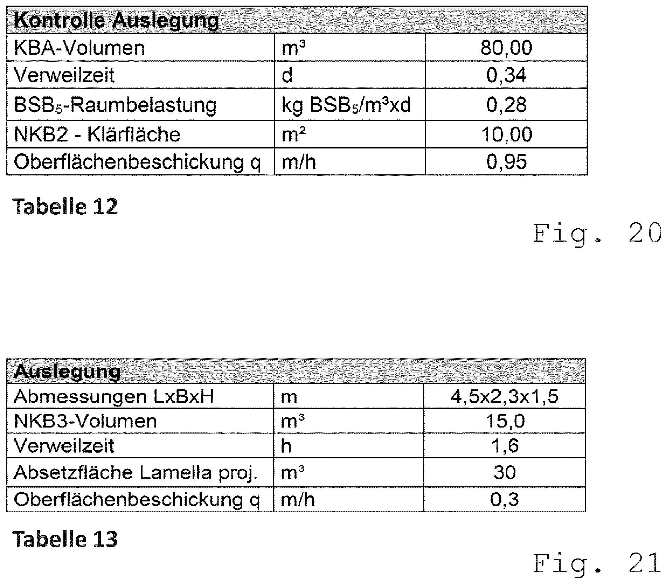

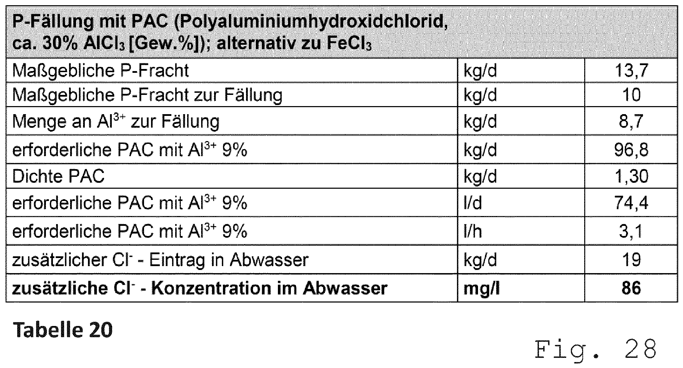

Als weitergehende Reinigungsstufe zur finalen Reduzierung des CSB-Wertes und ggf. der Korrektur des P_{ges}.-Gehaltes wird das Abwasser dem NKB 3 zugeführt, der als Absetzbehälter mit integriertem Lamellapacket ausgebildet ist (siehe Fig. 21). Das Lamellapacket hat die Aufgabe die Klärfläche durch schräg eingebrachte Platten zu erhöhen und den Rückhalt der feinen Flocken aus der Fällungsprozess zu verbessern. Dem Zulauf wird ein effektives Fällungsmittel zugegeben (siehe Fig. 28), um die für die Einleitung geforderte Werte (CSB, P_{ges}.) ggf. zu korrigieren.

Zum Abbau des persisten CSB-Gehalts wird dem Abwasser ein weiteres Fällmittel, wie beispielsweise PAC (Polyaluminiumhydroxidchlorid), zugegeben (siehe Fig. 28). Der anfallende Schlamm wird im Nachklärbecken über Schwerkraft abgeschieden und zum Nachgärer 1 abgezogen.

Zur Fällung/Flockung in der weitergehenden Abwasserreinigung zur sicheren Einhaltung der CSB/P - Einleitkonzentrationen eignen sich alternativ auch andere Chemikalien.

Nach Durchlaufen der Reinigung bzw. Gärresteaufbereitung können bspw. etwa 80.300 m³ Abwasser vorflutreif mit einem TS-Gehalt von 35 mg/l und einem CSB-Gehalt von 200 mg/l vorliegen. Die Einleitung kann über einen Pumpschacht in einen durch das Anlagengelände verlaufenden Regenwasserkanal erfolgen. Auf Grund betriebsbedingter Schwankungen (Schwankung Stoffeigenschaften Zuckerrüben, Diskontinuität Niederschlag zur Waschwasservorhaltung, Erhöhung Wasserzufuhr Dekanteranlage, Erhöhung Rückführung Überschussschlamm, usw.) kann sich die jährlich eingeleitete Abwassermenge verändern.

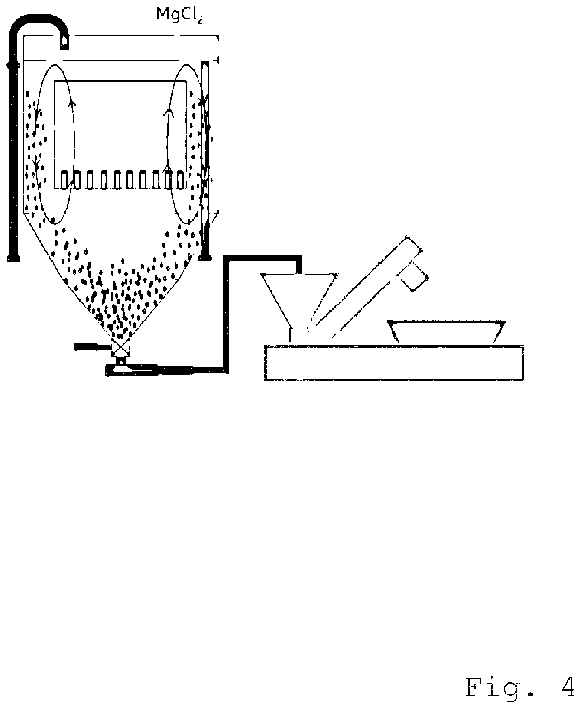

Eine weitere Behandlungsstufe umfasst das Ausfällen von Magnesium-Amonium-Phosphat (MAP) mittels eines sogenannten AirPrex®-Verfahrens (Fig. 4). Hierdurch lässt sich die biologische Phosphat-Elimination verbessern, wobei der Gärrest nach einem Fermenter in ein in Fig. 4 gezeigtes Reaktorsystem geführt und dort einer Luftströmung unterzogen wird. Durch Lufzufuhr erfolgt ein Ausgasen von CO₂ wodurch der ph-Wert des Gärrestschlammes deutlich ansteigt.

Zusätzlich wird Magnesiumchlorid (MGCL₂) zugeführt, was zur Bildung und Ausfällung von Magnesium-Amonium-Phosphat (MAP) führt. Die so entstandenen MAP-Kristalle können in dem Reaktor wachsen und setzen sich am Boden des Reaktorsystems aufgrund von Sedimentation ab. Anschließend können die MAP-Kristalle über ein Fördersystem einer Waschanlage zur Aufbereitung der Kristalle, beispielsweise als Düngemittel, zugeführt werden.

### Maßnahmen zur Vermeidung von Abwasser

Während der Rübenannahmekampagne kann verschmutztes Niederschlagswasser der Rübenannahmefläche im Absetzcontainer zwischengespeichert und zur Rübenwäsche genutzt werden.

Anfallendes überschüssiges verschmutztes Niederschlagswasser kann in den Nachgärer 3 abgesenkt werden.

Zum Kampagnenende wird die Fläche gereinigt. Nachfolgend kann der Absenkweg Richtung Regenrückhaltebecken geöffnet und das unverschmutzte Niederschlagswasser eingeleitet werden.

### Bestandteile der Gärrestaufbereitungsanlage

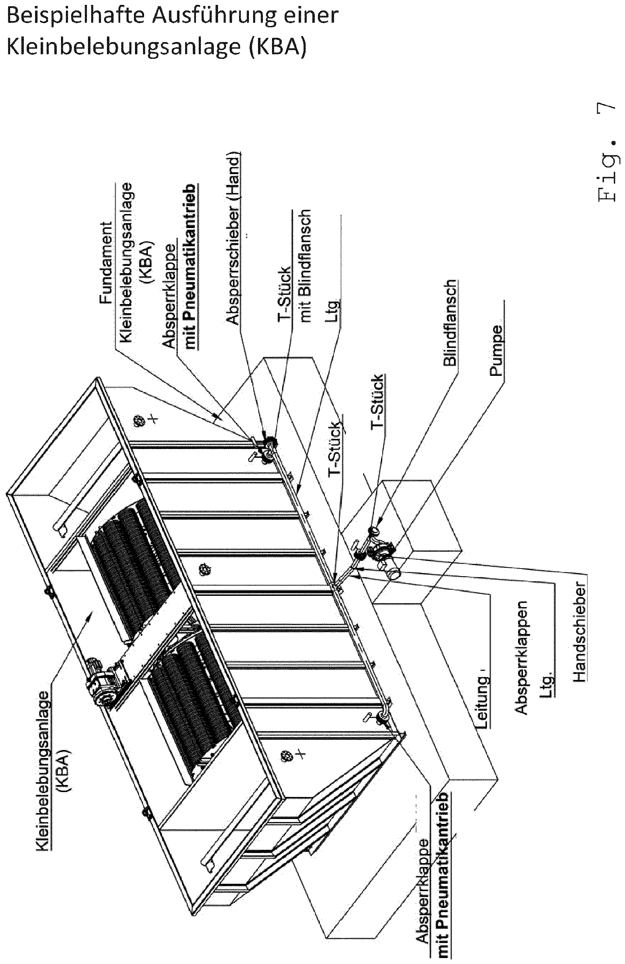

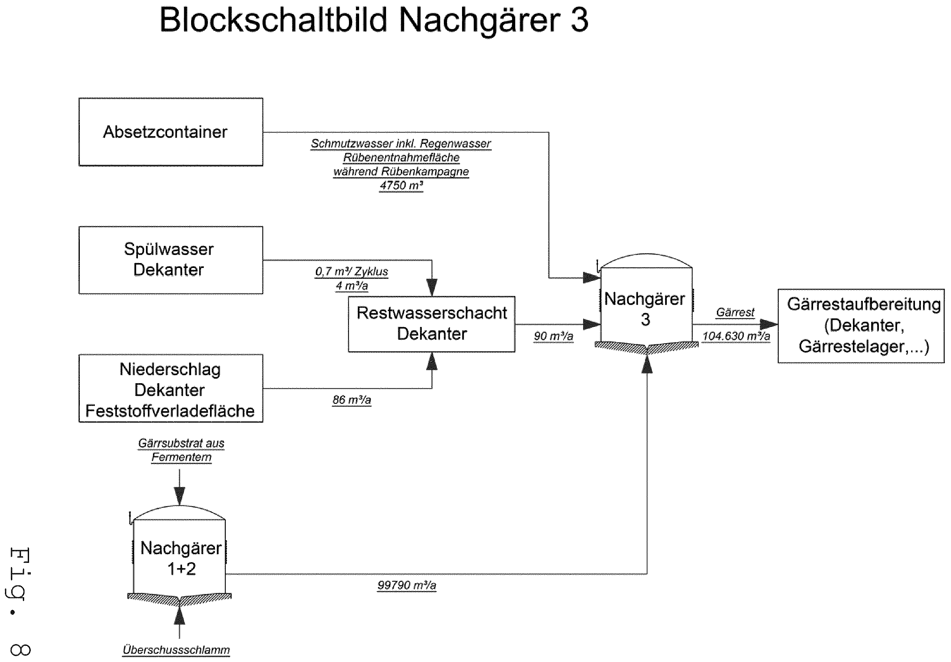

In den Fig. 7 und 8 sind Bestandteile der Gärrestaufbereitungsanlage im Detail dargestellt.In Fig. 7 ist eine beispielhafte Ausführung einer Kleinbelebungsanlage (KBA) in Containerbauweise gezeigt.

In Fig. 8 ist ein Blockschaltbild des Nachgärers 3 gezeigt. Dem Nachgärer 3 werden im Betrieb der Biogasanlage mit zusätzlicher Gärrestaufbereitung sowohl Schmutzwasser, inklusive Regenwasser, aus den Absetzcontainern, Spülwasser aus der Dekanteranlage sowie Niederschlagswasser aus der Verladefläche der Dekanteranlage und Gärreste aus den Nachgärern zugeführt. Dem Nachgärer 3, der als Vorstufe zur Gärrestaufbereitung dient, werden daher sämtliche anfallende Gärreste und Flüssigkeiten zugeführt.

Für die Pufferung von sowohl Überschussschlamm als auch Rübenmus stehen Lagertanks 1-8 zur Verfügung (siehe Fig. 3). Die Lagertanks sind über Rohre mit den Nachklärbecken, Kleinbelebungsanlage, Nachgärer, Fermenter/Biogasanlage (BGA) und die Versorgung mit Rübenmus sowie untereinander verbunden und können je nach Bedarf über Schieber, Pumpen und Ventile so geschaltet werden, dass entweder Überschussschlamm oder Rübenmus eingelagert bzw. ausgelagert wird. Ein Vermischung der beiden Substanzen in den Lagertanks findet nicht statt.

Der in den Figuren gezeigte Aufbau der Gesamtanlage zur Gärrestaufbereitung und Biogaserzeugung zeichnet sich dadurch aus, dass als Endprodukt vorflutreifes Abwasser bereitgestellt wird. Die Bereitstellung des Wassers erfolgt durch die vorgehend beschriebenen Reinigungsschritte, wobei die Dosierung und Auswahl der zugesetzten Mittel anhand des Ausführungsbeispiels für Zuckerrüben in den Tabellen angegeben ist. Bei anderen Substraten als Ausgangsprodukte für die Gärrestaufbereitung kann sich die Menge der zugesetzten Stoffe auch von den Beispielen unterscheiden.

## Patentansprüche

1. Verfahren zur Aufbereitung von Gärresten, die bei der Vergärung zur Biogaserzeugung anfallen, aufweisend wenigstens folgende Verfahrensschritte:
- Zuführen der anfallenden Gärreste aus einer vorgelagerten Gäreinrichtung zur Biogaserzeugung einer weiteren Nachgäreinrichtung,
- Nachgären der anfallenden Gärreste in der weiteren Nachgäreinrichtung,
- Zuführen der Gärreste aus der weiteren Nachgäreinrichtung einer Separiereinrichtung,
- Trennen der Feststoffe von dem Zentrat der Gärreste (flüssige Bestandteile) in der Separiereinrichtung,
- Zuführen des Zentrats einem Gärrestlager,
- Zuführen des Zentrats einem ersten Belebungsbecken,
- biologische Reinigung des Zentrats durch einen Belebtschlammprozess mittels Bakterien, wobei eine Nitrifikation und Denitrifikation bei einer intermittierenden Belüftung erfolgen, und wobei in der belüftete Phase Sauerstoff und in der unbelüfteten Phase eine externe Kohlenstoffquelle zugeführt werden,
- Zuführen des Belebtschlammes einem ersten Nachklärbecken, in dem eine Trennung des Belebtschlamms durch Separation erfolgt,
- Abführen des Klarfiltrats,
- Zuführen des Klarfiltrats einer Kleinbelebungsanlage mit integriertem zweitem Nachklärbecken, wobei durch einen weiteren Belebtschlammprozess mittels Bakterien eine biologische Reinigung und Durchmischung des Klarfiltrats unter Zuführung von Sauerstoff erfolgt,
- Zuführen des geklärten Abwassers einem dritten Nachklärbecken, in dem wenigstens ein Fällungsmittel und/oder Reaktionsmittel dem geklärten Abwasser zugeführt werden.

2. Verfahren nach Anspruch 1, wobei dem Klarfiltrat vor und/oder während dem Einleiten in die Kleinbelebungsanlage ein Fällungsmittel zugeführt wird.

3. Verfahren nach Anspruch 1 oder 2, wobei der bei der biologischen Reinigung im ersten Nachklärbecken, im zweiten Nachklärbecken und im dritten Nachklärbecken anfallende Überschussschlamm der vorgelagerten Gäreinrichtung zugeführt wird.

4. Verfahren nach einem der Ansprüche 1 bis 3, wobei den Gärresten vor der Trennung der Feststoffphase von dem Zentrat ein Flockungsmittel zugeführt wird.

5. Verfahren nach einem der Ansprüche 1 bis 4, wobei bei der Trennung der Feststoffphase von dem Zentrat Verdünnungswasser zugeführt wird.

6. Verfahren nach einem der Ansprüche 1 bis 5, wobei die belüftete Phase und die unbelüftete Phase jeweils 1 bis 2 Stunden, vorzugsweise 1,5 Stunden, aufrechterhalten werden.

7. Verfahren nach einem der Ansprüche 1 bis 6, wobei als Fällungsmittel Aluminiumchlorid (AlCl₃) oder Polyaluminiumhydroxidchlorid (PAC) und/oder als Flockungsmittel Polymere zugeführt werden.

8. Verfahren nach einem der Ansprüche 1 bis 7, wobei als Kohlenstoffquelle Glycerin zugeführt wird.

9. Anlage zur Aufbereitung von Gärresten, wobei die Anlage dazu ausgelegt und ausgebildet ist, ein Verfahren nach einem der Ansprüche 1 bis 8 durchzuführen, mindestens aufweisend
- eine weitere Nachgäreinrichtung,
- eine Separiereinrichtung zur Trennung der festen Phase vom Zentrat des Gärrests,
- ein Gärrestlager,
- ein erstes Belebungsbecken zur biologischen Reinigung des Zentrats,
- ein erstes Nachklärbecken zur Trennung des Belebtschlamms,
- eine Kleinbelebungsanlage mit integriertem zweitem Nachklärbecken zur weiteren biologischen Reinigung, und
- ein drittes Nachklärbecken zur Absetzung des Belebtschlamms.

10. System zur Aufbereitung von Gärresten, aufweisend Komponenten zur Durchführung der Verfahrensschritte der Ansprüche 1 bis 8.

11. Verwendung einer Anlage nach Anspruch 9 oder eines Systems nach Anspruch 10 zur Aufbereitung von Gärresten und/oder zur Abwasseraufbereitung.
